# EUROPEAN PATENT APPLICATION

(11) **EP 2 713 339 A1**
(43) Date of publication of application: **02.04.2014**
(21) Application number: 13185376.4
(22) Date of filing: 20.09.2013
(51) Int. Cl.: G06T 11/00

(54) **Image processing device, radiographic image capturing system, image processing program and image processing method**

(30) Priority: 28.09.2012 JP 2012218263
(71) Applicant: Fujifilm Corporation, Minato-ku Tokyo (JP)
(72) Inventor: Fukuda, Wataru, Kanagawa (JP)
(74) Representative: Klunker . Schmitt-Nilson . Hirsch

(57) **Abstract**

An image processing device (50) includes: acquisition means that acquire plural projection images captured by applying radiation from multiple directions, while moving radiation applying means (24), with respect to an object (W) between the radiation applying means and a radiation detector (42) and by detecting the radiation via the radiation detector; tomographic image generating means (66) that generate tomographic images of the object by reconstructing them from the acquired plural projection images; projection image mask generating means (68) that generate plural projection image masks for separating an object region and a direct incidence region of each of the plural projection images; tomographic image mask generating means (70) that reconstruct and generate, from the plural projection image masks, tomographic image masks for separating the object region and the direct incidence region of the tomographic images; and masking means (72) that perform masking by applying the tomographic image masks to the tomographic images.

## Description

### BACKGROUND OF THE INVENTION

### Field of the Invention

The present invention pertains to an image processing device, a radiographic image capturing system, an image processing program, and an image processing method. The present invention particularly relates to an image processing device that generates tomographic images from radiographic images captured by applying radiation from different directions with respect to an object, a radiographic image capturing system, an image processing program, and an image processing method.

### Description of the Related Art

Conventionally, radiographic image capturing apparatus that perform radiographic imaging for the purpose of medical diagnosis have been known. Mammography machines, which capture images of the breasts of a subject for the purpose of early detection of breast cancer and so forth, are an example of this type of radiographic image capturing apparatus. And in mammography machines, a technology that performs tomosynthesis imaging, which captures images of the breasts of a subject by applying radiation from multiple directions, is known.

Slice images of breasts acquired by tomosynthesis imaging are regarded as being particularly effective with respect to high-density breasts (dense breasts) (much mammary gland). One reason for this is that in a case where a dense breast is imaged from one direction, the transmittance of the radiation through the glandular and connective tissue is low, so there is the concern that a mass or the like, for example, will end up being hidden by the presence of the glandular and connective tissue and will not be reflected in the image, leading to misdiagnosis, but imaging a dense breast from multiple directions makes it easier for the mass or the like to be reflected in the image.

Tomosynthesis imaging produces tomographic images parallel to the object table surface at predetermined slice intervals by reconstructing the tomographic images from radiographic images captured by applying radiation from different directions with respect to an object.

Incidentally, in the case of displaying tomographic images, their value as images improves more (glare can be reduced, it becomes easier to see object regions, etc.) by blackly displaying direct incidence regions that do not include an image of the object. In a case where reversal processing that reverses tonal gradation (density) has been performed with respect to the tomographic images, the direct incidence regions become white and end up becoming glaringly bright, so there is also the demand to maintain the black state in regard to the direct incidence regions.

A technology that performs pixel padding value (PPV) processing with respect to a radiographic image captured by a mammography machine and masks the direct incidence region is known (e.g., see Japanese Patent Application Laid-open (JP-A) No. 2009-297393). A technology that obtains three-dimensional outline information by fitting a two-dimensional model conresponding to the sectional shape of an imaged site of an object within the boundary of a sinogram of a captured image and estimating two-dimensional outline information of the imaged site sticking out from incompletely captured images (captured images in which only part of the object is projected) on the basis of the two-dimensional model (fit curve) that has been fit is also known (e.g., see JP-A No. 2007-143954).

### SUMMARY OF THE INVENTION

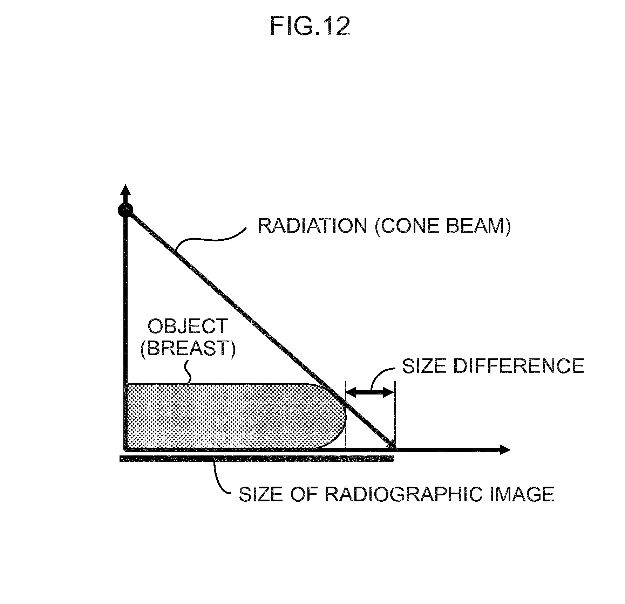

The technology described in JP-A No. 2009-297393 is a technology that generates radiographic image masks and masks direct incidence regions, but it is not a technology that generates tomographic image masks. As shown in FIG. 12, in tomosynthesis imaging, a cone beam of radiation is applied to an object to obtain radiographic images, but even if, for example, masks corresponding to radiographic images obtained when the radiation has been applied from a perpendicular direction with respect to the object (here, a breast) are generated, the sizes of the object region and the direct incidence region differ between those masks and masks corresponding to tomographic images reconstructed from the plural radiographic images, so the masks corresponding to the radiographic images cannot be applied as is to the tomographic images. The boundary data of the sinogram used in the technology of JP-A No. 2007-143954 are also boundary data in radiographic images and are not boundary data in tomographic images.

The present invention provides an image processing device that can implement, with good precision, masking with respect to tomographic images, a radiographic image capturing system, an image processing method, and an image processing program.

According to a first aspect of the invention, an image processing device includes: acquisition means that acquire plural projection images captured by applying radiation from multiple directions, while moving radiation applying means, with respect to an object between the radiation applying means and a radiation detector and by detecting the radiation via the radiation detector; tomographic image generating means that generate tomographic images of the object by reconstructing them from the acquired plural projection images; projection image mask generating means that generate plural projection image masks for separating an object region and a direct incidence region of each of the plural projection images, the object region being a region that includes an image of the object and the direct incidence region being a region that does not include an image of the object; tomographic image mask generating means that reconstruct and generate, from the plural projection image masks, tomographic image masks for separating the object region and the direct incidence region of the tomographic images; and masking means that perform masking by applying the tomographic image masks to the tomographic images.

Because the image processing device generates the tomographic image masks by reconstructing them from the projection images masks, tomographic image masks corresponding to the tomographic images can be obtained, and the image processing device can implement masking with good precision.

According to a second aspect of the invention, the image processing device may be configured that the tomographic image mask generating means generate plural tomographic image masks corresponding to the plural tomographic images included in a first tomographic image group when the plural tomographic images generated by the tomographic image generating means have been divided into the first tomographic image group at the radiation applying means side and a second tomographic image group at a radiation detector side, and in the case of performing masking with respect to the second tomographic image group, the masking means perform the masking by reversing the corresponding order of the plural tomographic image masks with respect to the second tomographic image group from the corresponding order of the plural tomographic images with respect to the first tomographic image group and applying the tomographic image masks.

Because of this, the amount of time for generating the tomographic images masks can be shortened, and by applying the tomographic image masks corresponding to the tomographic images of the first tomographic image group to the masking of the tomographic images of the second tomographic image group, the precision of the masking can be maintained compared to the case of applying the tomographic image masks corresponding to the tomographic images of the second tomographic image group to the masking of the tomographic images of the first tomographic image group.

According to a third aspect of the invention, the image processing device may be configured to further include image processing means which, in the case of performing image processing other than the masking with respect to the tomographic images, perform the image processing before the masking is performed.

By performing the image processing other than the masking before the masking is performed, the occurrence of artifacts can be suppressed.

According to a fourth aspect of the invention, the image processing device may be configured that the tomographic image mask generating means reconstruct and generate, from the projection image masks, the region, among the all regions of the tomographic image masks, where the applied regions of the radiation applied from the multiple directions coincide with one another.

The processing for generating the masks becomes faster compared to the case of performing the reconstruction in regard to the entire regions.

According to a fifth aspect of the invention, the image processing device may be configured that the tomographic image mask generating means perform the reconstruction of the tomographic image masks by performing an operation taking sections in which the plural projection image masks are the object region as the object region and taking a section in which at least one projection image mask of the plural projection image masks is the direct incidence region as the direct incidence region.

According to this configuration, the processing for generating the tomographic image masks becomes faster compared to the case of performing the reconstruction by performing addition.

According to a sixth aspect of the invention, the image processing device may be configured that the tomographic image mask generating means reconstruct and generate the tomographic image masks from plural reduced masks obtained by reducing the plural projection image masks, and the masking means perform the masking by restoring the tomographic image masks to their original sizes.

By reconstructing and generating the tomographic image masks from the reduced masks obtained by reducing the projection image masks, the processing for generating the tomographic image masks becomes faster compared to the case of reconstructing the tomographic image masks without reducing the projection image masks.

According to a seventh aspect of the invention, the image processing device may be configured to further include storage means that store, as data in one-dimensional form or function form, the tomographic image masks generated by the tomographic image mask generating means, and the masking means perform the masking using the tomographic image masks represented by the data stored in the storage means.

Storage capacity can be reduced compared to the case of storing the image information of the tomographic image masks in bitmap form.

According to an eighth aspect of the invention, a radiographic image capturing system includes: a radiographic image capturing apparatus that captures plural radiographic images by applying radiation from multiple directions, while moving the radiation applying means, with respect to an object on the radiation detector by the radiation applying means disposed in opposition to the radiation detector and by detecting the radiation by the radiation detector; and the image processing device pertaining to any of the first aspect to the seventh aspect of the invention.

According to a ninth aspect of the invention, an image processing program for causing a computer to function as: acquisition means that acquire plural projection images captured by applying radiation from multiple directions, while moving radiation applying means, with respect to an object on the radiation detector by the radiation applying means disposed in opposition to a radiation detector and by detecting the radiation via the radiation detector; tomographic image generating means that reconstruct and generate tomographic images of the object from the acquired plural projection images; projection image mask generating means that generate plural projection image masks for separating an object region and a direct incidence region of each of the plural projection images, the object region being a region that includes an image of the object and the direct incidence region being a region that does not include an image of the object; tomographic image mask generating means that reconstruct and generate, from the plural projection image masks, tomographic image masks for separating the object region and the direct incidence region of the tomographic images; and masking means that perform masking by applying the tomographic image masks to the tomographic images.

According to a tenth aspect of the invention, an image processing method includes: acquiring plural projection images captured by applying radiation from multiple directions, while moving radiation applying means, with respect to an object on the radiation detector by the radiation applying means disposed in opposition to a radiation detector and by detecting the radiation by the radiation detector; reconstructing and generating tomographic images of the object from the acquired plural projection images; generating plural projection image masks for separating an object region and a direct incidence region of each of the plural projection images, the object region being a region that includes an image of the object and the direct incidence region being a region that does not include an image of the object; reconstructing and generating, from the plural projection image masks, tomographic image masks for separating the object region and the direct incidence region of the tomographic images; and performing masking by applying the tomographic image masks to the tomographic images.

The radiographic image capturing system, the image processing program, and the image processing method described above also act in the same way as the image processing device pertaining to the first aspect, so they can implement masking with good precision.

As described above, according to the present invention, masking with respect to tomographic images can be implemented with good precision.

### BRIEF DESCRIPTION OF THE DRAWINGS

An embodiment of the present invention will be described in detail based on the following figures, wherein:
FIG. 1 is a plan view showing an example of a configuration of a radiographic image capturing apparatus of the embodiment;
FIG. 2 is a view showing an example of a configuration during imaging by the radiographic image capturing apparatus of the embodiment;
FIG. 3 is an explanatory drawing for describing the imaging by the radiographic image capturing apparatus of the embodiment;
FIG. 4 is a block diagram showing an example of a configuration of a radiographic image capturing system of the embodiment;
FIG. 5 is a drawing schematically showing, in a projection image, an object region (in this embodiment, a section including a breast N) and a direct incidence region that does not include the object;
FIG. 6 is a flowchart showing an example of a flow of tomographic image output processing of the embodiment;
FIG. 7A is a drawing showing an example of a tomographic image before application of a tomographic image mask;
FIG. 7B is a drawing showing an example of a tomographic image after application of a tomographic image mask (i.e., after masking);
FIG. 8 is a flowchart showing an example of a flow of tomographic image output processing where image processing is implemented before masking;
FIG. 9A is a drawing schematically showing image information having binary pixel values;
FIG. 9B is a schematic drawing for describing one-dimensional image information;
FIG. 10 is an explanatory diagram in a case where tomographic images are divided into two groups and tomographic image masks corresponding to one group are also used in the masking of the other group;
FIG. 11 is an explanatory diagram describing a region where applied ranges of radiation applied at different angles with respect to an object coincide with one another; and
FIG. 12 is an explanatory diagram describing the size difference between a radiographic image obtained by applying a cone beam of radiation to an object and a tomographic image.

### DETAILED DESCRIPTION OF THE INVENTION

An embodiment of the present invention will be described in detail below with reference to the drawings. This embodiment is not intended to limit the present invention.

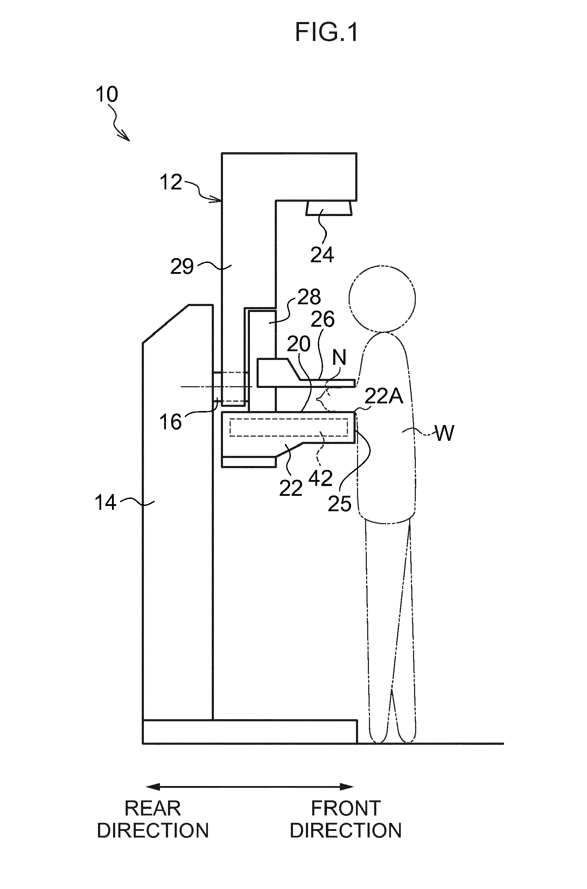

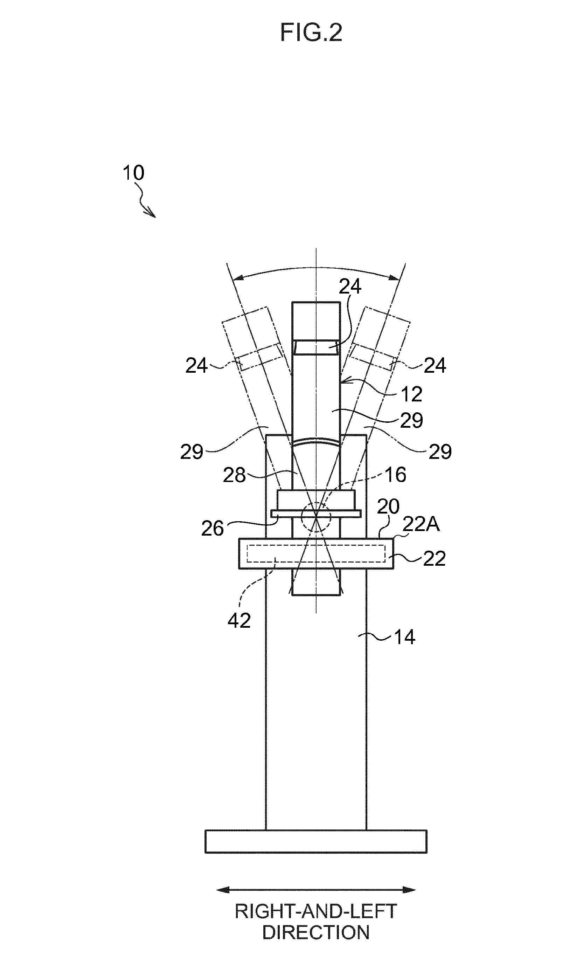

As shown in FIG. 1 to FIG. 3, a radiographic image capturing apparatus 10 of the present embodiment is an apparatus that uses radiation (e.g., X-rays) to capture an image of a breast N of a subject W in an erect state in which the subject W is standing, and is a mammography machine, for example. Below, the near side close to the subject W in a case where the subject W is facing the radiographic image capturing apparatus 10 during imaging will be called the apparatus front side of the radiographic image capturing apparatus 10, the far side away from the subject W in a case where the subject W is facing the radiographic image capturing apparatus 10 will be called the apparatus back side of the radiographic image capturing apparatus 10, and the right-and-left direction of the subject W in a case where the subject W is facing the radiographic image capturing apparatus 10 will be called the apparatus right-and-left direction of the radiographic image capturing apparatus 10 (see the arrows in FIG. 1 and FIG. 2).

The imaging target of the radiographic image capturing apparatus 10 is not limited to the breast N and may also be another site of the body, or an object, for example. The radiographic image capturing apparatus 10 may also be an apparatus that captures an image of the breast N of the subject W in a seated state in which the subject W is seated in a chair (including a wheelchair) or the like. It suffices for the radiographic image capturing apparatus 10 to be an apparatus that can capture individual images of the right and left breasts N of the subject W at least in a state in which the upper half of the body of the subject W is erect.

As shown in FIG. 1, the radiographic image capturing apparatus 10 is equipped with a measurement unit 12, which is substantially C-shaped as seen in a side view and is disposed on the apparatus front side, and a base unit 14, which supports the measurement unit 12 from the apparatus back side.

The measurement unit 12 is configured to include an object table 22 on which is formed a planar object table surface 20 with which the breast N of the subject W in the erect state comes into contact, a compression paddle 26 for compressing the breast N between the compression paddle 26 and the object table surface 20 of the object table 22, and a holder 28 that supports the object table 22 and the compression paddle 26. A member that allows radiation to pass through it is used for the compression paddle 26.

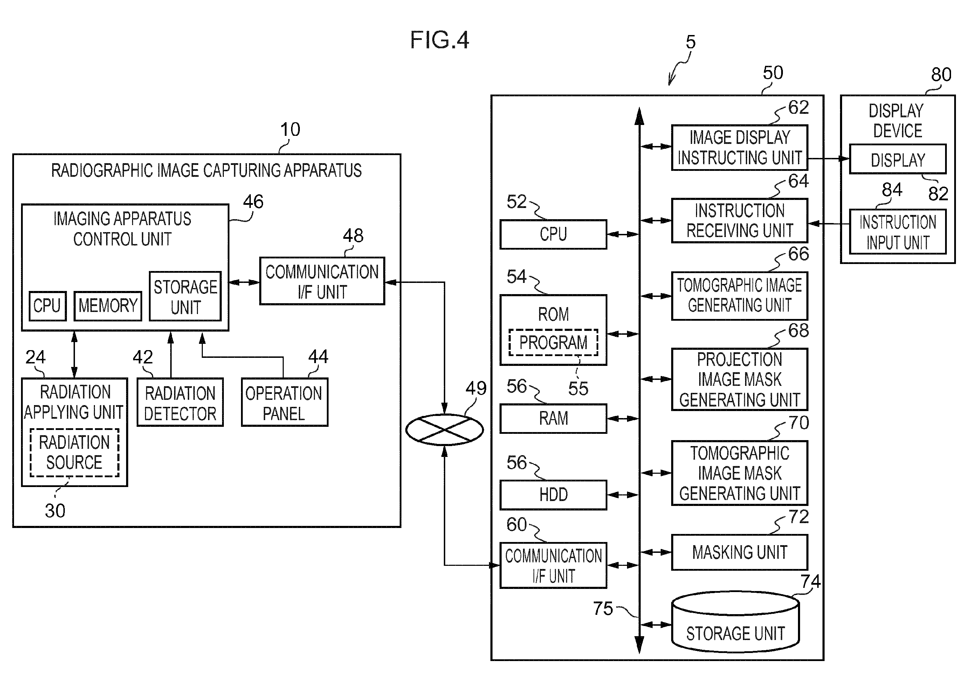

The measurement unit 12 is provided with a radiation applying unit 24, which is disposed with a radiation source 30 (see FIG. 4) such as a tube and applies radiation for testing from the radiation source 30 toward the object table surface 20, and a support 29, which is separate from the holder 28 and supports the radiation applying unit 24.

A rotating shaft 16 supported in such a way that it can rotate in the base unit 14 is disposed in the measurement unit 12. The rotating shaft 16 is fixed with respect to the support 29, so that the rotating shaft 16 and the support 29 rotate integrally.

The rotating shaft 16 is switchable between a state in which it is coupled to and rotates integrally with the holder 28 and a state in which it is decoupled from the holder 28 and idles. Specifically, gears are disposed on the rotating shaft 16 and the holder 28, and the gears are switched between a state in which they are meshed with one another and a state in which they are not meshed with one another.

A variety of machine elements can be used to switch between transmitting and not transmitting the rotational force of the rotating shaft 16.

The holder 28 supports the object table 22 in such a way that the object table surface 20 and the radiation applying unit 24 are a predetermined distance away from one another and slidably holds the compression paddle 26 in such a way that the distance between the compression paddle 26 and the object table surface 20 can be varied.

The object table surface 20 with which the breast N comes into contact is made of carbon, for example, from the standpoints of radiation transmittance and strength. A radiation detector 42, to which the radiation that has passed through the breast N and the object table surface 20 is applied and which detects the radiation, is disposed inside the object table 22. The radiation detected by the radiation detector 42 is made visible so that a radiographic image is generated.

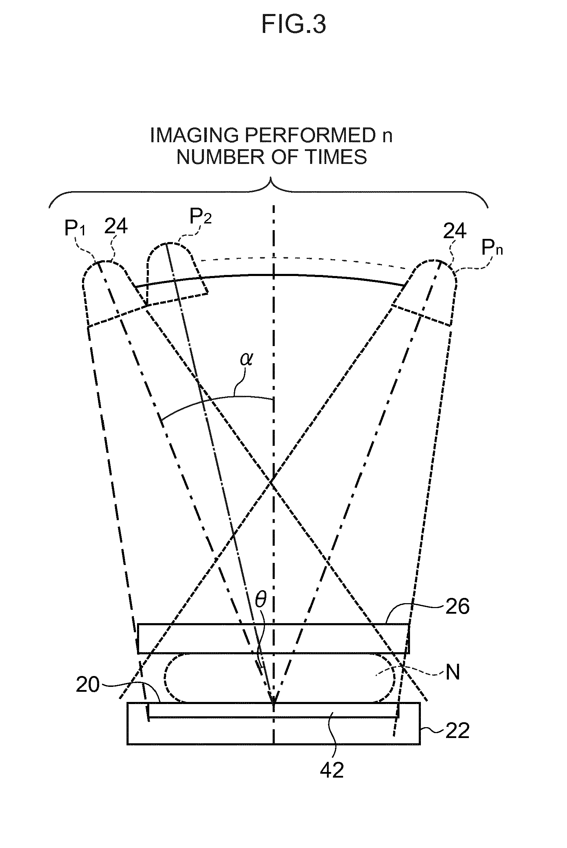

The radiographic image capturing apparatus 10 of the present embodiment is an apparatus that can at least perform imaging by applying radiation from multiple directions with respect to the breast N serving as the object. FIG. 2 shows orientations of the radiographic image capturing apparatus 10 during imaging, and FIG. 3 shows positions of the radiation applying unit 24 during imaging. As shown in FIG. 2 and FIG. 3, the radiographic image capturing apparatus 10 performs imaging by tilting the support 29 that supports the radiation applying unit 24 and supports the object table 22 via the holder 28.

As shown in FIG. 3, in a case where the radiographic image capturing apparatus 10 performs imaging (tomosynthesis imaging) by applying radiation from multiple directions to the breast N, the rotating shaft 16 idles with respect to the holder 28 so that the object table 22 and the compression paddle 26 do not move, and the support 29 rotates so that just the radiation applying unit 24 moves in a circular arc. In the present embodiment, as shown in FIG. 3, the imaging position is moved by a predetermined angle θ at a time from angle α, and imaging is performed in n number of positions P1 to Pn of the radiation applying unit 24.

The radiographic image capturing apparatus 10 of the present embodiment can perform both craniocaudal (CC) imaging and mediolateral-oblique (MLO) imaging with respect to the breast N. During CC imaging, the orientation of the holder 28 is adjusted to a state in which the object table surface 20 faces up, and the orientation of the support 29 is adjusted to a state in which the radiation applying unit 24 is positioned directly over the object table surface 20. Because of this, the radiation is applied from the radiation applying unit 24 to the breast N, from the head of the subject W in the erect state toward the feet, and CC imaging is performed. During MLO imaging, generally the orientation of the holder 28 is adjusted to a state in which, compared to during CC imaging, the object table 22 is rotated 45° or more and less than 90°, and the armpit of the subject W is positioned in such a way as to be rest against a side wall corner portion 22A on the apparatus front side of the object table 22. Because of this, the radiation is applied from the radiation applying unit 24 to the breast N, outward from the axial center of the torso of the subject W, and MLO imaging is performed.

A chest wall surface 35, with which the section of the chest on the underside of the breast N of the subject W is brought into contact during imaging, is formed on the surface of the object table 22 on the apparatus front side. The chest wall surface 25 is planar.

FIG. 4 is a block diagram showing an example of a configuration of a radiographic image capturing system 5 of the present embodiment.

The radiographic image capturing system 5 of the present embodiment is configured to include the radiographic image capturing apparatus 10, an image processing device 50, and a display device 80.

The radiographic image capturing apparatus 10 is configured to include the radiation applying unit 24, the radiation detector 42, an operation panel 44, an imaging apparatus control unit 46, and a communication interface (I/F) unit 48.

The imaging apparatus control unit 46 has the function of controlling the operation of the entire radiographic image capturing apparatus 10 and is configured to include a central processing unit (CPU), a memory including a read-only memory (ROM) and a random access memory (RAM), and a nonvolatile storage unit comprising a hard disk drive (HDD) or flash memory. The imaging apparatus control unit 46 is connected to the radiation applying unit 24, the radiation detector 42, the operation panel 44, and the communication I/F unit 48.

When the imaging apparatus control unit 46 receives an instruction from an operator via the operation panel 44 (exposure switch) to apply radiation, the imaging apparatus control unit 46 causes the radiation to be applied from the radiation source 30 disposed in the radiation applying unit 24 to the object table surface 20 in accordance with an imaging menu (details described below) that has been set on the basis of designated exposure conditions. In the present embodiment, the radiation source 30 applies a cone beam of radiation (e.g., a conical X-ray beam).

The radiation detector 42 receives the radiation, which carries image information, records the image information, and outputs the recorded image information. The radiation detector 42 is, for example, configured as a flat panel detector (FPD) that is disposed with a radiation-sensitive layer, converts radiation into digital data, and outputs the digital data. When the radiation is applied to the radiation detector 42, the radiation detector 42 outputs the image information representing the radiographic image to the imaging apparatus control unit 46. In the present embodiment, the radiation detector 42 receives the radiation that has passed through the breast N and obtains the image information representing the radiographic image.

The operation panel 44 has the function of allowing the operator to set various types of operation information, such as exposure conditions and orientation information, and various types of operation instructions.

The exposure conditions set via the operation panel 44 include information such as tube voltage, tube current, exposure time, and orientation information. The orientation information set via the operation panel 44 includes information expressing the imaging position (imaging orientation, angle) in the case of performing imaging from multiple directions with respect to the breast N.

The various types of operation information, such as the exposure conditions and the orientation information, and various types of operation instructions may be set by the operator via the operation panel 44, or may be obtained from another control device (a radiology information system (RIS), which is a system that manages information relating to treatments and diagnoses using radiation), or may be stored beforehand in a storage unit.

When the various types of information are set via the operation panel 44, the imaging apparatus control unit 46 executes radiographic image capture by causing the radiation to be applied from the radiation applying unit 24 to the imaging site (the breast N) of the subject W in accordance with the imaging menu that has been set on the basis of the various types of information that have been set. In the case of performing imaging by applying radiation from multiple directions to the breast N, the imaging apparatus control unit 46 adjusts the orientation of the holder 28 to a state in which the object table surface 20 faces up and adjusts also the orientation of the support 29 to a state in which the radiation applying unit 24 is positioned directly over the object table surface 20. Then, as shown in FIG. 3, the imaging apparatus control unit 46 rotates the support 29 to move the radiation applying unit 24 by angle θ at a time from angle α in a circular arc and, on the basis of the imaging conditions, causes the radiation to be applied individually at different angles with respect to the object table surface 20 from the radiation source 30 disposed in the radiation applying unit 24. Because of this, n number of radiographic images are obtained.

The communication I/F unit 48 is a communication interface having the function of transmitting and receiving, via a network 49, the captured radiographic images and various types of information between the radiographic image capturing apparatus 10 and the image processing device 50.

The image processing device 50 has the function of generating tomographic images reconstructed from the radiographic images acquired from the radiographic image capturing apparatus 10. The image processing device 50 also has the functioning of performing, with respect to the radiographic images, image processing for a doctor or the like to view an object of concern such as a mass. Below, the person-such as a doctor-who views the captured radiographic images and generated tomographic images and diagnoses tumors and so forth will be called a user, and the radiographic images obtained as a result of the radiation detector 42 detecting the radiation by tomosynthesis imaging in the radiographic image capturing apparatus 10 will be called projection images.

The image processing device 50 is configured to include a CPU 52, a ROM 52, a RAM 56, a HDD 58, a communication I/F unit 60, an image display instructing unit 62, an instruction receiving unit 64, a tomographic image generating unit 66, a projection image mask generating unit 68, a tomographic image mask generating unit 70, a masking unit 72, and a storage unit 74. These are connected to one another such that they can transmit and receive information and so forth between one another, via a bus 75 such as a control bus or a data bus.

The CPU 52 controls the entire image processing device 50. Specifically, the CPU 52 controls the image processing device 50 by executing a program 55 stored in the ROM 54. In the present embodiment, the program 55 is stored beforehand, but the program 55 is not limited to this. The program 55 may also be stored beforehand in a recording medium or the like such as a CD-ROM or removable disk and installed in the ROM 54 or the like from the recording medium, or may be installed in the ROM 54 or the like from an external device via a communication line such as the Internet. The RAM 56 ensures a region for work when the CPU 52 executes the program 55. The HDD 58 stores and holds various types of data.

The communication I/F unit 60 is a communication interface having the function of transmitting and receiving, via the network 49, the captured radiographic images and various types of information between the image processing device 50 and the radiographic image capturing apparatus 10.

The image display instructing unit 62 has the function of instructing a display 82 of the display device 80 to display the radiographic images.

The display device 80 of the present embodiment has the function of displaying the captured radiographic images and is configured to include the display 82, on which the radiographic images are displayed, and an instruction input unit 84. The instruction input unit 84 has the function of allowing the user (e.g., a doctor) wanting to view an object of concern such as a mass to input instructions relating to the display of the radiographic images. Examples of the instruction input unit 84 include a touch panel display, a keyboard, and a mouse.

The instruction receiving unit 64 has the function of receiving the instructions from the user that have been input via the instruction input unit 84 of the display device 80.

The tomographic image generating unit 66 has the function of acquiring the plural projection images obtained by tomosynthesis imaging, reconstructing tomographic images of the object from the acquired plural projection images, and generating tomographic images that are parallel to the object table surface 20 at predetermined slice intervals. In the present embodiment, "parallel" also includes substantially parallel.

The tomographic image generating unit 66 generates the tomographic images at predetermined slice intervals from the plural projection images captured by moving the radiation source 30 to the positions of P1, P2, P3, and so on to Pn. The position at which an object of concern is projected on the radiographic images differs depending on the angle at which the radiation source 30 applies the radiation. Thus, the tomographic image generating unit 66 acquires the imaging conditions at the time the radiographic images were captured from the radiographic image capturing apparatus 10, calculates the amount of movement of the object of concern between the radiographic images on the basis of the applied angles of the radiation included in the imaging conditions, and reconstructs the tomographic images on the basis of a publicly known reconstruction method such as shift addition. Shift addition is a publicly known method that shifts and adds together the projection images such that the target section is emphasized and the peripheral section is defocused.

Conventionally publicly known CT reconstruction methods other than shift addition can also be used for the reconstruction processing method. For example, filtered back projection (FBP), which is a representative CT reconstruction method, can be used. FBP is a reconstruction technique in which filtered back projection is expanded by taking parallel plane tomographic scanning in tomographic imaging as part of cone beam CT scanning. If projection images are simply back-projected (added), noise (blurring) arises in the peripheral sections, so in FBP, the projection images are back-projected after a filter has been applied to the projection images to obtain images close to the original target. The iterative reconstruction method described in JP-ANo. 2011-125698 can also be used. This iterative reconstruction method is a reconstruction method for CT, but like FBP, it can also be applied to reconstruction during tomosynthesis imaging.

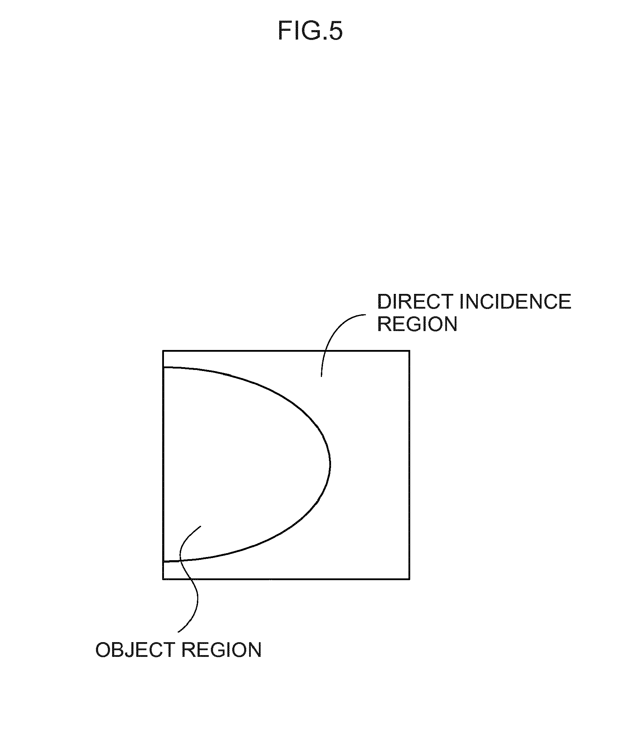

The projection image mask generating unit 68 generates mask images (called "projection image masks" below) of the plural projection images captured in the plural positions described above. Here, as shown in the schematic drawing of FIG. 5, the projection image masks are images for separating object regions and direct incidence regions of the projection images. The object regions are regions that include the object (in the present embodiment, sections including the breast N), and the direct incidence regions are regions that do not include the object. For example, the projection image masks can be expressed as binary image information where 0 represents the pixel values of the object regions and 1 represents the pixel values of the direct incidence regions. The projection image masks may also be expressed as binary image information where 1 represents the pixel values of the object regions and 0 represents the pixel values of the direct incidence regions. As for the method of generating the projection image masks, the projection image mask generating unit 68 can generate the projection image masks by performing pixel padding value (PPV) processing using a threshold value, for example. The projection image mask generating unit 68 may also use the publicly known technology described in JP-A No. 2009-297393. The projection image mask generating unit 68 may also generate the projection image masks by using the technology described in JP-A No. 6-251149 to cluster the projection images into outline regions (object regions) and background regions (direct incidence regions). The projection image mask generating unit 68 may also generate the projection image masks by using the technology described in JP-A No. 2004-283281 to extract the edge of the object by obtaining and binarizing outline data corresponding to changes in luminance between plural pixels.

The methods exemplified above are known less as techniques for tomosynthesis imaging and more as methods used to extract an object in radiographic images obtained by ordinary two-dimensional imaging (ordinary imaging that applies radiation to, and captures an image of, an object from a fixed position without moving the radiation source 30). The dose of radiation in tomosynthesis imaging is lower than the dose of radiation during two-dimensional imaging, so although projection images obtained by tomosynthesis imaging are not the exact same type of image as radiographic images obtained by two-dimensional imaging, the characteristics of both are the same, so there are no problems whatsoever in using, for the processing of projection images obtained by tomosynthesis imaging, a method used for radiographic images obtained by two-dimensional imaging.

The tomographic image mask generating unit 70 generates mask images (called "tomographic image masks" below) of the tomographic images. Like the projection image masks, the tomographic image masks are images for separating object regions and direct incidence regions of the tomographic images. For example, the tomographic image masks can be expressed as binary image information where 0 represents the pixel values of the object regions and 1 represents the pixel values of the direct incidence regions. In the present embodiment, the masking unit 72 implements masking that masks the direct incidence regions by applying the tomographic image masks, so the tomographic image masks can also be generated as mask images for masking the direct incidence regions.

In the present embodiment, the tomographic image mask generating unit 70 generates the tomographic image masks corresponding to the tomographic images by reconstructing them from the projection image masks generated by the projection image mask generating unit 68. For example, the tomographic image mask generating unit 70 applies a publicly known reconstruction processing method used when generating tomographic images from projection images also to the generation of the tomographic image masks to generate the tomographic image masks. For example, the tomographic image mask generating unit 70 may use shift addition, FBP, or iterative reconstruction. The tomographic image mask generating unit 70 generates plural tomographic image masks corresponding to the plural tomographic images generated in the tomographic image generating unit 66.

The masking unit 72 performs masking that masks the direct incidence regions of the tomographic images (e.g., filling in with black, for example, predetermined pixel values) by applying the tomographic image masks generated by the tomographic image mask generating unit 70 to the tomographic images generated by the tomographic image generating unit 66. The CPU 52 outputs the image information of the tomographic images masked in the masking unit 72 to the image display instructing unit 62 so that the image information can be displayed on the display 82 of the display device 80. Or, the masking unit 72 can also output the image information of the masked tomographic images directly to the image display instructing unit 62 without involving the CPU 52.

The tomographic image generating unit 66, the projection image mask generating unit 68, the tomographic image mask generating unit 70, and the masking unit 72 can be realized by hardware configured by general electronic circuits, application-specific integrated circuits (ASIC), or field-programmable gate arrays (FPGA).

The storage unit 74 has the function of storing image information and so forth expressing the projection images captured by the radiographic image capturing apparatus 10, the tomographic images, the projection image masks, and the tomographic image masks. The storage unit 74 is a large-capacity storage device such as a hard disk, for example. In the present embodiment, the storage unit 74 also stores the imaging conditions (the angles at which the radiation was applied, etc.) when radiographic image capture was performed by the radiographic image capturing apparatus 10.

Next, the action of the radiographic image capturing system 5 of the present embodiment will be described with reference to the drawings.

In the case of performing radiographic image capture, when an imaging menu is set, the radiographic image capturing apparatus 10 executes imaging in accordance with the imaging menu.

In a case where an instruction to perform imaging by applying the radiation from multiple directions to the breast N has been input, as shown in FIG. 2, the radiographic image capturing apparatus 10 adjusts the orientation of the holder 28 to a state in which the object table surface 20 faces up and also adjusts the orientation of the support 29 to a state in which the radiation applying unit 24 is positioned directly over the object table surface 20.

The subject W moves her breast N into contact with the object table surface 20 of the radiographic image capturing apparatus 10. When the operator performs an operation with respect to the operation panel 44 to instruct the start of compression in this state, the compression paddle 26 moves toward the object table surface 20.

In a case where an instruction to perform imaging by applying the radiation from multiple directions to the breast N has been input to the operation panel 44 in this state, as shown in FIG. 3, the radiographic image capturing apparatus 10 rotates just the support 29 to move the radiation applying unit 24 by predetermined angle θ at a time from angle α in a circular arc and applies the radiation based on the imaging conditions in n number of positions P1 to Pn of the radiation applying unit 24. The beams of radiation individually applied from the radiation applying unit 24 pass through the breast N and thereafter reach the radiation detector 42.

When the beams of radiation are applied to the radiation detector 42, the radiation detector 42 outputs to the imaging apparatus control unit 46 the sets of image information representing the projection images resulting from the applied radiation. In a case where, as described above, application of the radiation has been performed in n number of positions P1 to Pn of the radiation applying unit 24, the radiation detector 42 outputs sets of image information corresponding to the n number of projection images to the imaging apparatus control unit 46.

The imaging apparatus control unit 46 outputs the input sets of image information to the image processing device 50. In a case where, as described above, application of the radiation has been performed in n number of positions P1 to Pn of the radiation applying unit 24, the CPU of the imaging apparatus control unit 46 outputs sets of image information corresponding to the n number of projection images to the image processing device 50.

The image processing device 50 reconstructs tomographic images from the n number of projection images acquired from the radiographic image capturing apparatus 10, performs masking by applying the separately generated tomographic image masks to the tomographic images, and performs tomographic image output processing that causes the tomographic images after the masking to be displayed on the display device 80.

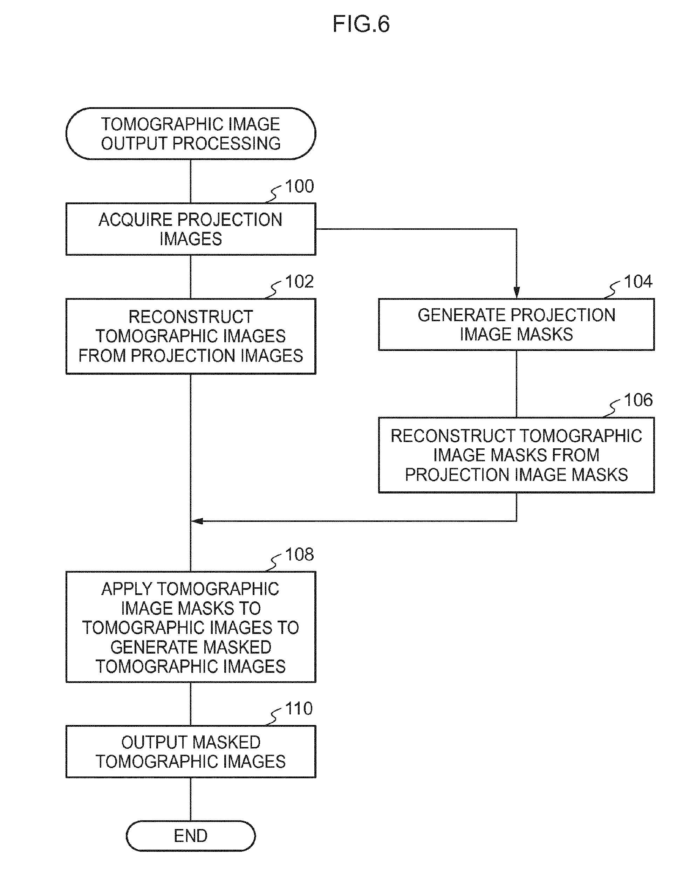

FIG. 6 is a flowchart showing an example of a flow of the tomographic image output processing executed in the image processing device 50 of the present embodiment. This processing is executed as a result of the control program 55 stored in the ROM 54 being processing by the CPU 52.

In step 100, the CPU 52 acquires the sets of image information of the plural (here, the n number of) projection images from the radiographic image capturing apparatus 10.

In step 102, the CPU 52 controls the tomographic image generating unit 66 to reconstruct tomographic images from the plural projection images.

In step 104, the CPU 52 controls the projection image mask generating unit 68 to generate projection image masks of the projection images.

In step 106, the CPU 52 controls the tomographic image mask generating unit 70 to reconstruct tomographic image masks from the generated projection image masks to generate tomographic image masks corresponding to the reconstructed and generated tomographic images.

In step 108, the CPU 52 controls the masking unit 72 to provide the generated tomographic image masks to the corresponding tomographic images, perform masking, and generate masked tomographic images.

In step 110, the CPU 52 outputs the image information of the masked tomographic images to the image display instructing unit 62 and causes the masked tomographic images to be displayed on the display 82.

The processing of step 102 and the processing of step 104 and step 106 may be performed in parallel, or the processing of step 104 and step 106 may be performed after the processing of step 102, or the processing of step 102 may be performed after the processing of step 104 and step 106.

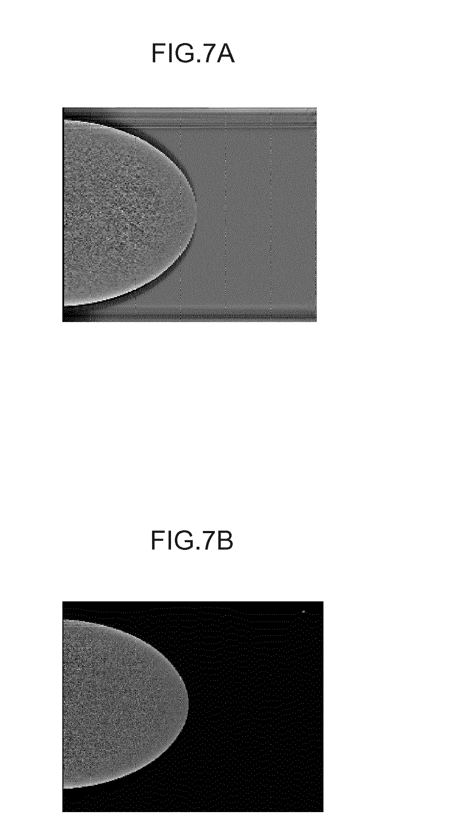

FIG. 7A shows an example of a tomographic image before application of a tomographic image mask, and FIG. 7B shows an example of a tomographic image after application of a tomographic image mask (i.e., after masking). Because the direct incidence region of the tomographic image can be masked in this way, glare when displaying the tomographic image can be reduced and it also becomes easier to see the object (object of concern).

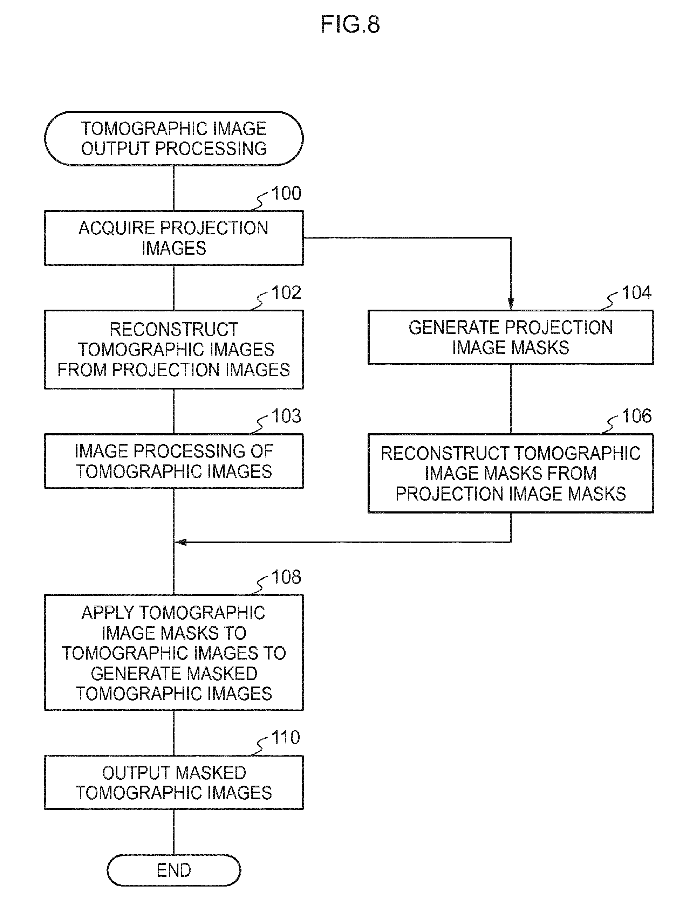

In a case where image processing other than the masking that applies the tomographic image masks to the tomographic images is to be performed with respect to the tomographic images, such as enhancement, noise removal, compression, or tone conversion, it is preferred that this image processing be performed before the masking in step 108 such as shown in step 103 in FIG. 8.

This is because when masking is implemented with respect to the tomographic images, a sharp edge appears in the boundary sections between the masked sections and the other sections, and if image processing such as exemplified above is performed in this state, there is the potential for artifacts to occur in the tomographic images.

As described above, in the radiographic image capturing system 5 of the present embodiment, the radiographic image capturing apparatus 10 applies radiation from different directions to the breast N of the subject W and captures plural projection images by tomosynthesis imaging. The image processing device 50 acquires the captured plural projection images and stores them in the storage unit 74. The image processing device 50 of the present embodiment reconstructs plural tomographic images from the plural projection images, generates plural projection image masks, and stores the plural tomographic images and the plural projection image masks in the storage unit 74. The image processing device 50 also reconstructs tomographic image masks corresponding to the tomographic images from the plural projection image masks and stores the tomographic image masks in the storage unit 74. Then, the image processing device 50 performs masking by applying the tomographic image masks to the tomographic images.

Because the image processing device 50 performs masking by using the projection image masks reconstructed from the projection images to reconstruct the tomographic image masks and applying the tomographic image masks to the tomographic images, masking with respect to the tomographic images can be performed with good precision.

The action of the image processing device 50 is not limited to the above.

For example, the image processing device 50 may also be configured to reduce the projection image masks generated in the projection image mask generating unit 68 and use the reduced projection image masks to reconstruct reduced tomographic image masks in the tomographic image mask generating unit 70. The image processing device 50 stores the image information of the reduced tomographic image masks in the storage unit. Then, the masking unit 72 reads the reduced tomographic image masks, restores them to their original sizes, and performs masking by applying the restored tomographic image masks to the tomographic images.

Image reconstruction requires linear processing time with respect to each pixel of the image, so by performing reconstruction from reduced tomographic image masks, reconstruction can be performed faster than in the case of performing reconstruction from same-size projection image masks that are not reduced. The storage capacity needed to store the tomographic image masks can also be reduced.

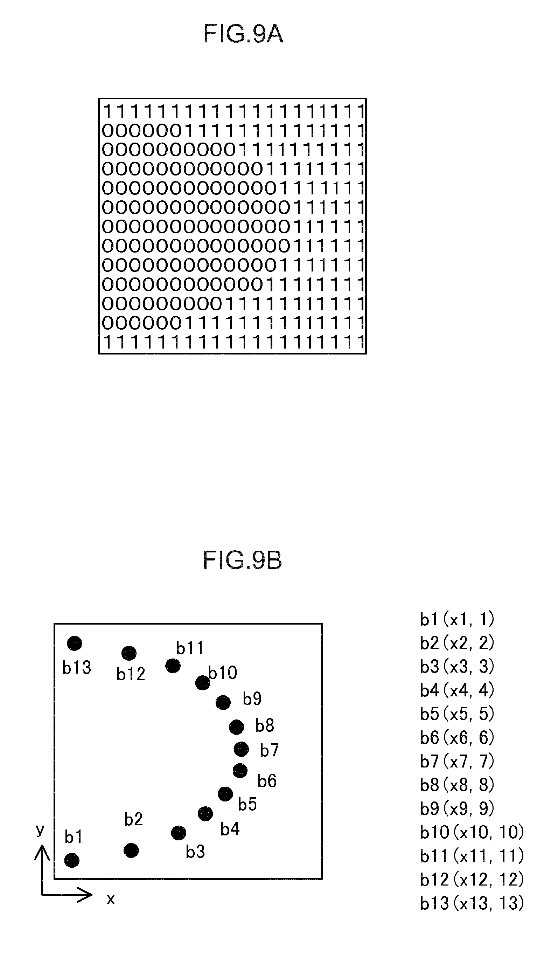

Further, as shown in the schematic drawing of FIG. 9A, the image information of the projection image masks and tomographic image masks can also be stored as two-dimensional information (bitmap data) in which pixel-unit pixel values (here, the binary values of 0 and 1 are exemplified) are arrayed. Alternatively, as shown in the schematic drawing of FIG. 9B, the image information of the projection image masks and tomographic image masks can also be stored as one-dimensional information in which x-axis coordinate values x1 to x13 on an outline of the object region or the direct incidence region on an x-y plane are arrayed in the order of y-axis coordinate values 1 to 13. By storing the masks as one-dimensional information in this way, the necessary storage capacity can be reduced, the amount of time it takes to write and read the image information is also shortened, and processing becomes faster. In particular, the number of tomographic images is greater than the number of projection images, so by storing the tomographic image masks corresponding to the tomographic images in one-dimensional form, the storage capacity needed to store the tomographic image masks is significantly reduced.

Moreover, the image processing device 50 may also be configured to generate functions expressing the outlines of the object regions or the direct incidence regions of the projection image masks and tomographic image masks and store the masks in function form. More specifically, the image processing device 50 can perform least square fitting (function approximation by the least square method) of a function model, for example, with respect to the projection image masks and tomographic image masks to thereby can find the value of each parameter of the function model and generate functions expressing the masks. Even by storing the masks in function form in this way, the necessary storage capacity can be reduced.

In a case where the image processing device 50 has stored the projection image masks in one-dimensional form or function form, when the tomographic image mask generating unit 70 generates the tomographic image masks, the tomographic image mask generating unit 70 can expand the image information into two-dimensional image information and use the two-dimensional image information for the reconstruction. Further, in a case where the image processing device 50 has stored the tomographic image masks in one-dimensional form or function form, in a case where the masking unit 72 performs masking, the masking unit 72 can expand the image information into two-dimensional image information and use the two-dimensional image information for the masking.

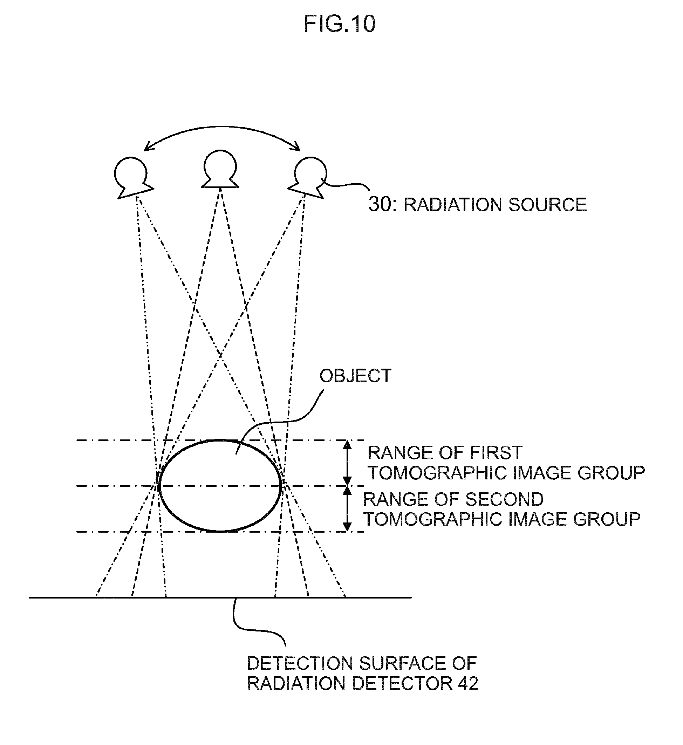

Further, instead of the tomographic image mask generating unit 70 generating tomographic image masks corresponding to all the tomographic images, as shown in FIG. 10, in a case where the plural tomographic images generated by the tomographic image generating unit 66 have been divided into a first tomographic image group on the radiation source 30 side and a second tomographic image group on the radiation detector 42 side, the tomographic image mask generating unit 70 may also generate only tomographic image masks corresponding to the tomographic images of the first tomographic image group. At this time, the plural tomographic images may be divided such that the number of tomographic images included in the second tomographic image group is the same as the number of tomographic images included in the first tomographic image group.

The masking unit 72 uses the tomographic image masks corresponding to the tomographic images of the first tomographic image group to implement masking with respect to the tomographic images of both the first tomographic image group and the second tomographic image group.

Specifically, with respect to the tomographic images of the first tomographic image group, the masking unit 72 performs masking by applying the plural tomographic image masks generated by the tomographic image mask generating unit 70 without changing the corresponding order of the corresponding tomographic image masks. In the case of performing masking with respect to the second tomographic image group, the masking unit 72 performs the masking by reversing the corresponding order of the plural tomographic image masks with respect to the second tomographic image group from the corresponding order of the plural tomographic image masks with respect to the first tomographic image group and applying the tomographic image masks.

For example, let it be assumed that six tomographic images have been generated, and let those six tomographic images be called D1, D2, D3, D4, D5, and D6 in order beginning with the tomographic image on the side close to the radiation source 30. The tomographic images D1, D2, and D3 on the radiation source 30 side are divided as the tomographic images of the first tomographic image group, and the tomographic images D4, D5, and D6 on the radiation detector 42 side are divided as the tomographic images of the second tomographic image group. The tomographic image mask generating unit 70 generates tomographic image masks M1, M2, and M3 corresponding to the tomographic images D1, D2, and D3. In a case where the masking unit 72 performs masking with respect to the tomographic images D1, D2, and D3 of the first tomographic image group, the masking unit 72 implements the masking by applying, without changing the corresponding order of, the tomographic image masks M1, M2, and M3. That is, the masking unit 72 implements the masking by applying M1 to D1, applying M2 to D2, and applying M3 to D3. In a case where the masking unit 72 performs masking with respect to the tomographic images D4, D5, and D6 of the second tomographic image group, the masking unit 72 reverses the corresponding order of the tomographic image masks M1, M2, and M3 with respect to the tomographic images D4, D5, and D6 of the second tomographic image group from the corresponding order with respect to the first tomographic image group and applies the tomographic image masks. That is, the masking unit 72 implements the masking by applying M3 to D4, applying M2 to D5, and applying M1 to D6.

As shown in FIG. 10, because the radiation is applied from the radiation source 30, the radiation becomes applied in a higher density on the radiation source 30 side. Consequently, a more real state is reflected in the first tomographic image group than in the second tomographic image group. Thus, masking can be performed efficiently and with high precision by applying the tomographic image masks of the first tomographic image group also as the tomographic image masks of the second tomographic image group as described above.

Moreover, the tomographic image mask generating unit 70 does not have to implement reconstruction in regard to all regions when generating the tomographic image masks. That is, the tomographic image mask generating unit 70 may also be configured to reconstruct and generate partial regions of the all regions of the tomographic image masks from the projection image masks.

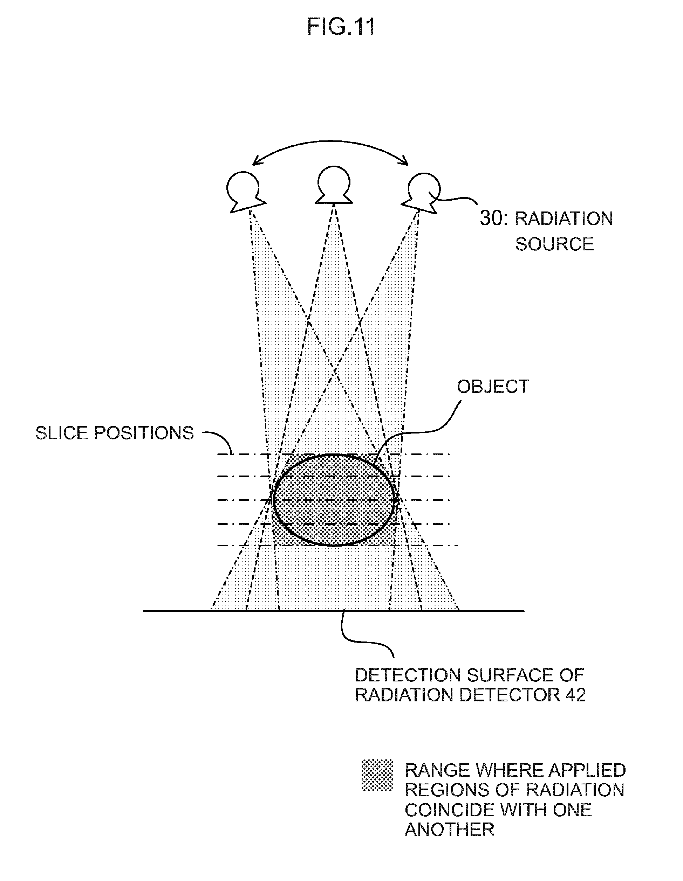

More specifically, as shown in the schematic drawing of FIG. 11 for example, the tomographic image mask generating unit 70 may be configured to implement the reconstruction taking as a target only the region where the applied ranges of the radiation applied from the different directions coincide with one another. This is because regions where the applied ranges of the radiation do not coincide can be judged as being direct incidence regions outside the object region even without performing reconstruction. The tomographic image mask generating unit 70 determines, on the basis of the imaging conditions, the region where the applied ranges of the radiation coincide with one another and the regions where the applied ranges of the radiation do not coincide with one another, implements the reconstruction taking as a target only the region where the applied ranges coincide with one another, and generates the tomographic image masks of the tomographic images. The tomographic image mask generating unit 70 processes the regions where the applied ranges do not coincide with one another as direct incidence regions. Because of this, the processing for generating the tomographic image masks is made faster.

Moreover, general reconstruction methods such as shift addition and FBP perform reconstruction by adding plural images, but when reconstructing the projection image masks to generate the tomographic image masks, the tomographic image mask generating unit 70 may also reconstruct the tomographic image masks using the method described below rather than reconstructing the tomographic image masks by simply adding the projection image masks and dividing them by the number of tomographic image masks that have been added like in ordinary tomographic image reconstruction.

For example, in a state in which the plural projection image masks are superimposed on top of one another, the tomographic image mask generating unit 70 performs the reconstruction of the tomographic image masks by performing an operation taking sections where the plural projection image masks are the object regions as the object regions and taking a section where at least one projection image mask of the plural projection image masks is the direct incidence region as the direct incidence region. This is equivalent to a logical OR operation when 0 expresses the object region of each projection image mask and 1 expresses the direct incidence region. However, which numerical values express the object regions and the direct incidence regions are not particularly limited, and 1 may express the object region of each projection image mask and 0 may express the direct incidence region. In this case also, it suffices to perform an operation taking sections where the plural projection image masks are the object regions as the object regions and taking a section where at least one projection image mask of the plural projection image masks is the direct incidence region as the direct incidence region. In the case of performing reconstruction with this method, division is unnecessary.

By performing processing in this way, the processing for generating the tomographic image masks is made faster than in the case of simply adding all the pixel values to reconstruct and generate the tomographic image masks.

Moreover, in the above embodiment, an example where the tomographic image mask generating unit 70 generates the tomographic image masks by reconstructing the tomographic image masks from the projection image masks using a publicly known reconstruction technique was described, but in addition to this, the tomographic image mask generating unit 70 can also generate the tomographic image masks using the method described below.

For example, the tomographic image mask generating unit 70 uses the projection image masks generated on the basis of the projection images obtained by applying the radiation from a direction perpendicular with respect to the object (the detection surface of the radiation detector 42), enlarges or reduces the object regions in correspondence to each tomographic image in such a way that the size of the object regions of the projection image masks become sizes corresponding to the separately generated tomographic images, takes the regions outside the object regions after enlargement or reduction as the direct incidence regions, and thereby generates the tomographic image masks. The enlargement factor or reduction factor when generating the tomographic image masks can be set beforehand per position in the depth direction of the tomographic image masks (the direction perpendicular with respect to the detection surface of the radiation detector 42) in accordance with the cone angle, the type of object, or the slide intervals. There are many cases where the shape of the object (in the present embodiment, the breast N) is not a pure circular shape or oval shape, and sometimes the shape of the object regions and the shape of the direct incidence regions differ slightly from how they are actually if they are simply enlarged or reduced, but the tomographic image masks can be generated faster compared to the method that generates the tomographic image masks by reconstructing them from the projection image masks.

The tomographic image mask generating unit 70 may also generate the tomographic image masks without using the projection image masks. For example, like the technique by which the projection image mask generating unit 68 generates the projection image masks, the tomographic image mask generating unit 70 can also generate the tomographic image masks by analyzing the tomographic images generated by the tomographic image generating unit 66. That is, the tomographic image mask generating unit 70 can generate the tomographic image masks by performing pixel padding value (PPV) processing using a threshold value in the tomographic images (e.g., taking regions equal to or less than the threshold value as the direct incidence regions). The tomographic image mask generating unit 70 may also use the publicly known technology described in JP-A No. 2009-297393 mentioned above. The tomographic image mask generating unit 70 may also generate the tomographic image masks by using the technology described in JP-A No. 6-251149 to cluster the tomographic images into outline regions (object regions) and background regions (direct incidence regions). Moreover, the tomographic image mask generating unit 70 may also generate the tomographic image masks by using the technology described in JP-A No. 2004-283281 to extract the edge of the object by obtaining and binarizing outline data corresponding to changes in luminance between plural pixels.

The number of tomographic images generated by the tomographic image generating unit 66 is greater than the number of projection images, so it takes time if the tomographic image mask generating unit 70 analyzes the individual tomographic images to generate the tomographic image masks as described above. Further, sometimes artifacts arise when the tomographic image generating unit 66 reconstructs the tomographic images, and in a case where the tomographic image mask generating unit 70 analyzes the tomographic images to generate the tomographic image masks, it is necessary to generate the tomographic image masks taking into consideration the effect of such artifacts, and because of this, sometimes it takes time for the tomographic image mask generating unit 70 to perform the analysis. However, masking can be performed with high precision by applying the tomographic image masks generated using this technique to the tomographic images.

Further, in the present embodiment, the image processing device 50 generates the tomographic images and projection image masks from the projection images stored in the storage unit 74 of the image processing device 50, but the image processing device 50 is not limited to this and may also be configured to generate the tomographic images and tomographic image masks from projection images received from outside via the network 49 or the like.

Further, in the present embodiment, a case where the present invention is applied to the generation of tomographic images of projection images captured by a mammography machine was described, but the present invention is not limited to this and may also be applied to the generation of tomographic images of projection images captured by other radiographic image capturing apparatus.

Further, the radiation used in tomosynthesis imaging is not particularly limited, and X-rays and gamma rays can be applied.

In addition, the configurations of the radiographic image capturing system 5, the radiographic image capturing apparatus 10, the image processing device 50, and the display device 80 described in the present embodiment are examples and, needless to say, can be changed in accordance with the situation without departing from the gist of the present invention.

Further, in the present embodiment, an example where the tomographic image generating unit 66, the projection image mask generating unit 68, the tomographic image mask generating unit 70, and the masking unit 72 are realized by hardware (e.g., hardware configured by general electronic circuits, ASIC, or FPGA) was described, but their functions may also be realized as a result of the CPU 52 executing programs.

Further, the flow of the tomographic image output processing described in the present embodiment is also an example and, needless to say, can be changed in accordance with the situation without departing from the gist of the present invention.

## Claims

1. An image processing device (50) comprising:
acquisition means that acquire plural projection images captured by applying radiation from multiple directions, while moving radiation applying means (24), with respect to an object (W) between the radiation applying means and a radiation detector (42) and by detecting the radiation via the radiation detector;
tomographic image generating means (66) that generate tomographic images of the object by reconstructing them from the acquired plural projection images;
projection image mask generating means (68) that generate plural projection image masks for separating an object region and a direct incidence region of each of the plural projection images, the object region being a region that includes an image of the object and the direct incidence region being a region that does not include an image of the object;
tomographic image mask generating means (70) that reconstruct and generate, from the plural projection image masks, tomographic image masks for separating the object region and the direct incidence region of the tomographic images; and
masking means (72) that perform masking by applying the tomographic image masks to the tomographic images.

2. The image processing device according to claim 1, wherein
the tomographic image mask generating means generate plural tomographic image masks corresponding to the plural tomographic images included in a first tomographic image group when the plural tomographic images generated by the tomographic image generating means have been divided into the first tomographic image group at a radiation applying means side and a second tomographic image group at a radiation detector side, and
in the case of performing masking with respect to the second tomographic image group, the masking means perform the masking by reversing the corresponding order of the plural tomographic image masks with respect to the second tomographic image group from the corresponding order of the plural tomographic images with respect to the first tomographic image group and applying the tomographic image masks.

3. The image processing device according to claim 1 or 2, further comprising image processing means which, in the case of performing image processing other than the masking with respect to the tomographic images, perform the image processing before the masking is performed.

4. The image processing device according to any one of claims 1 to 3, wherein the tomographic image mask generating means reconstruct and generate, from the projection image masks, a region, among all regions of the tomographic image masks, in which applied regions of the radiation applied from the multiple directions coincide with one another.

5. The image processing device according to any one of claims 1 to 3, wherein the tomographic image mask generating means perform the reconstruction of the tomographic image masks by performing an operation taking sections in which the plural projection image masks are the object region as the object region and taking a section in which at least one projection image mask of the plural projection image masks is the direct incidence region as the direct incidence region.

6. The image processing device according to any one of claims 1 to 5, wherein
the tomographic image mask generating means reconstruct and generate the tomographic image masks from plural reduced masks obtained by reducing the plural projection image masks, and
the masking means perform the masking by restoring the tomographic image masks to their original sizes.

7. The image processing device according to any one of claims 1 to 6, further comprising storage means (74) that store, as data in one-dimensional form or function form, the tomographic image masks generated by the tomographic image mask generating means,
wherein the masking means perform the masking using the tomographic image masks represented by the data stored in the storage means.

8. A radiographic image capturing system (5) comprising:
a radiographic image capturing apparatus (10) that captures plural radiographic images by applying radiation by the radiation applying means disposed in opposition to the radiation detector from multiple directions, while moving the radiation applying means, with respect to the object on the radiation detector and by detecting the radiation via the radiation detector; and
the image processing device (50) according to any one of claims 1 to 7 that generates tomographic images from the plural radiographic images captured by the radiographic image capturing apparatus and performs masking.

9. An image processing program for causing a computer to function as each means of the image processing device according to any one of claims 1 to 7.

10. An image processing method comprising:
acquiring plural projection images (100) captured by applying radiation from multiple directions, while moving radiation applying means, with respect to an object on a radiation detector by the radiation applying means disposed in opposition the a radiation detector and by detecting the radiation via the radiation detector;
reconstructing and generating tomographic images of the object from the acquired plural projection images (102);
generating plural projection image masks for separating an object region and a direct incidence region of each of the plural projection images (104), the object region being a region that includes an image of the object and the direct incidence region being a region that does not include an image of the object;
reconstructing and generating, from the plural projection image masks, tomographic image masks for separating the object region and the direct incidence region of the tomographic images (106); and
performing masking by applying the tomographic image masks to the tomographic images (108).
